(19) Europäisches Patentamt
European Patent Office
Office européen des brevets

(11) Publication number: 0 174 046 A2

(12) **EUROPEAN PATENT APPLICATION**

(21) Application number: 85201358.0

(22) Date of filing: 26.08.85

(51) Int. Cl.[4]: C 07 C 131/00
C 07 C 153/00, C 07 D 249/08
A 01 N 37/52, A 01 N 37/50
A 01 N 43/653

(30) Priority: 07.09.84 GB 8422701

(43) Date of publication of application:
12.03.86 Bulletin 86/11

(84) Designated Contracting States:
AT BE CH DE FR GB IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Munro, David
40 Harvesters Way
Maidstone Kent(GB)

(72) Inventor: Clark, Michael Thomas
"Torcross" Wises Lane Borden
Sittingbourne Kent(GB)

(74) Representative: Hunter, Keith Roger Ian et al,
4 York Road
London SE1 7NA(GB)

(54) **Ether herbicides.**

(57) Oximino diphenyl ether derivatives having the general formula I:–

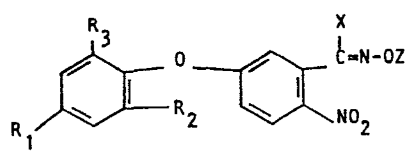

(1)

wherein $R_1$ represents a halogen atom or a haloalkyl group; $R_2$ and $R_3$, which may be the same or different, each independently represents a hydrogen or halogen atom or a nitro, cyano or haloalkyl group;

X represents a cyano or triazole group or group $SR_4$ or $NR_5R_6$ in which $R_4$ represents an optionally substituted alkyl, aryl, aralkyl, alkoxycarbonylalkyl or acyl group; $R_5$ represents an optionally substituted alkyl or aralkyl group and $R_6$ represents an optionally substituted alkyl, aralkyl or alkoxy group or the groups $R_5$ and $R_6$ together jointly represent an alkylene group;

and Z represents a hydrogen atom or an optionally substituted alkyl, cycloalkyl, alkenyl, aralkyl, alkoxycarbonylalkyl, or acyl group. The preparation of such compounds; herbicidal compositions containing them and their herbicidal use.

EP 0 174 046 A2

K 1963

ETHER HERBICIDES

This invention relates to certain oximino diphenyl ether derivatives, the preparation of such compounds, herbicidal compositions containing them, and to their use in combating undesired plant growth.

Certain diphenyl ethers are known to be effective herbicides; for example UK Patent Application No. 2049695 describes diphenyl ether alkanoic acids and their use as herbicides. It has now been found that useful herbicidal activity is present in diphenyl ethers bearing an oximino substituent.

Accordingly, the present invention provides oximino diphenyl ether derivatives having the general formula I:

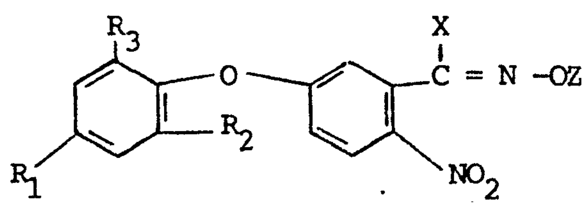

(I)

wherein $R_1$ represents a halogen, preferably chlorine, atom or a haloalkyl group; $R_2$ and $R_3$, which may be the same or different, each independently represents a hydrogen or halogen, preferably chlorine, atom or a nitro, cyano or haloalkyl group; X represents a cyano or triazole group or a group $SR_4$ or $NR_5R_6$ in which $R_4$ represents an optionally substituted alkyl, aryl, aralkyl, alkoxycarbonylalkyl or acyl group; $R_5$ represents an optionally substituted alkyl or aralkyl group and $R_6$ represents an optionally substituted alkyl, aralkyl or alkoxy group, or the

groups $R_5$ and $R_6$ together jointly represent an alkylene group; and Z represents a hydrogen atom or an optionally substituted alkyl, cycloalkyl, alkenyl, aralkyl, alkoxycarbonylalkyl or acyl group.

When any of the above substituents represents or contains an alkyl or haloalkyl group, this suitably contains up to 6, preferably up to 4, carbon atoms, and the halogen atom in the haloalkyl group is suitably fluorine or chlorine, with trifluoromethyl being the preferred haloalkyl group. When any of the substituents represent or contain an aryl group, this is preferably a phenyl group, which may optionally be substituted. When the aforesaid groups are optionally substituted, suitable substituents include a halogen, especially chlorine, atom and a cyano group. When $R_5$ and $R_6$ together represent an alkylene group, this may be straight or branched chain, and preferably contains from 3 to 8, especially 4 or 5, carbon atoms. When either of X or Z represents an acyl group, this may be derived from any organic acid, e.g. a carboxylic, sulphonic, phosphonic or carbamic acid.

Preferred compounds are those in which $R_1$ represents a haloalkyl, especially trifluoromethyl, group; $R_2$ represents a halogen, especially chlorine, atom; and $R_3$ represents a hydrogen atom. X preferably represents a cyano or triazole group or the group $SR_4$ wherein $R_4$ is an alkyl group containing up to 4 carbon atoms, especially methyl, a phenyl group, or an alkanoyl or alkoxycarbonylalkyl group of up to 8 carbon atoms, especially acetyl or ethoxycarbonylmethyl, or a group $NR_5R_6$ wherein $R_5$ and $R_6$ each represents an alkyl group containing up to 4 carbon atoms, especially methyl, or $R_5$ and $R_6$ jointly represent an alkylene group containing from 3 to 8 carbon atoms, especially a tetra- or penta-methylene group. Z preferably represents a hydrogen atom, an alkyl group containing up to 4 carbon atoms, especially methyl, optionally substituted by a cyano or phenyl group, an alkanoyl, alkylcarbamoyl or alkylsulphonyl group of up to 6 carbon atoms, especially acetyl, methylcarbamoyl or methylsulphonyl, or an alkoxycarbonylalkyl group of up to 8

carbon atoms, especially methoxycarbonylmethyl.

The invention also provides a process for the preparation of ethers as defined above, which comprises reacting a halo aldoxime of formula II:-

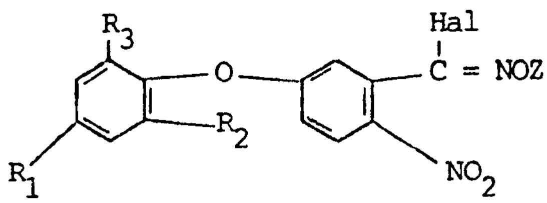

(II)

with a compound of formula XH, wherein $R_1$, $R_2$, $R_3$, X and Z are as defined above and Hal is halogen, in the presence of a base. A suitable base is a trialkylamine, such as triethylamine, and and the reaction is preferably carried out in an organic solvent such as ether. The reaction can conveniently be carried out at ambient temperature (25°C). When X represents $SR_4$ or $NR_5R_6$, then Z is preferably a hydrogen atom. The product of this reaction is then an oxime compound of formula I wherein Z is a hydrogen atom; when other derivatives of formula I are required, these may be obtained by further reacting said oxime with a compound of formula Hal Z (wherein Z is other than hydrogen) in the presence of an alkali metal hydride such as sodium hydride. This further reaction is preferably carried out in a polar organic solvent, such as tetrahydrofuran, and can conveniently be effected under reflux.

The compounds of general formula I have been found to show interesting activity as herbicides. Accordingly, the invention further provides a herbicidal composition comprising a compound of formula I as defined above in association with at least one carrier, and a method of making such a composition which comprises bringing a compound of formula I into association with at least one carrier.

The invention also provides the use of such a compound or composition according to the invention as a herbicide. Further, in accordance with the invention there is provided a method of combating undesired plant growth at a locus by treating the locus with a compound or composition according to the invention. Application to the locus may be pre-emergence or post-emergence.

The dosage of active ingredient used may, for example, be from 0.05 to 4kg/ha. A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this

process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually

containing ½-10% w of active ingredient. Granules are usually prepared to have a size between 10 and 100 BS mesh (1.676 - 0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension conentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The invention is illustrated in the following examples.

Example 1 Preparation of alpha-methylthio-3-(2-chloro-4-trifluoromethylphenoxy)-6-nitro-benzaldoxime

a) Nitration of 3-(2-chloro-4-trifluoromethylphenoxy)-benzoic acid.

The above starting compound (20g) was mixed with methylene dichloride (40ml) and concentrated sulphuric acid (80ml), and potassium nitrate (6.5g) added over 30 minutes with cooling to maintain the temperature at 0°C. The resulting reaction mixture was poured into ice-water, extracted with chloroform, and the solvent removed to give a pale yellow oil which was recrystallised to yield colourless crystals m.pt. 149-155°C.

b) The product of a) (9g) was dissolved in benzene (50ml) and thionyl chloride (3.3g) added dropwise with stirring and reflux under dry nitrogen for 1 hour. The solvent was removed to give a colourless oil which was used directly in the next stage.

c) The product of b) (9.5) dissolved in acetone (50ml) was reacted with triphenyl phosphine (13g) and bis(triphenylphosphine) copper borohydride (15.5g), and stirred at ambient temperature for 1 hour. The reaction mixture was filtered and solvent removed to yield an orange oil, which was dissolved in chloroform and stirred with cuprous chloride for 1 hour, filtered and solvent removed. The resulting oil was chromatographically purified and recrystallized to yield colourless crystals, m.pt. 95-96°C of 3-(2-chloro-4-trifluoromethylphenoxy)-6-nitro benzaldehyde.

Elemental Analysis:- Found C=48.9; H=1.9; N=3.9%

Calc. for $C_{14}H_7NClF_3O_4$ C=48.6; H=2.05; N=4.05%

d) Hydroxylamine hydrochloride (8.2g) was dissolved in water (30ml) and sodium acetate added (9.8g), followed by the benzaldehyde product of c) above (5g) and ethanol. The mixture was stirred under nitorgen at reflux until a clear solution was obtained, and reflux then continued for a

further 30 minutes. Methylene dichloride and water (800ml; 50/50) was added, the organic layer separated, washed, dried and the solvent removed to yield a yellow oil which was chromatographically purified and recrystallised to yield colourless crystals, m.pt. 120°C.

e) N-chlorosuccinimide (1.1g) was added to a stirred solution of the benzaldoxime of d) (3g) in dimethyl formamide (10ml) at 25°C, and the mixture heated to 50-60°C to initiate reaction. The temperature was then maintained around 50°C by the rate of addition of the remainder of the chlorosuccinimide (4.4g) and by heating/cooling. The product was poured into ice-water, extracted x2 with ether, and the organic layer washed and dried. Removal of solvent gave the alpha-chloro benzaldoxime as a colourless oil.

f) The alpha-chloro benzaldoxime of e) (4g) was dissolved in ether (20ml) and a solution of triethylamine (1.4g) in ether (20ml) added dropwise under nitrogen at ambient temperature, giving an immediate precipitate of triethylamine hydrochloride. Methyl mercaptan was bubbled through the solution at ambient temperature with stirring for 1 hour. Ether and water (500ml; 50/50) was added, the organic layer separated, washed and dried. The solvent was removed and the product chromatographically purified to yield the desired final product, m.pt. 180°C.

Elemental Analysis:- Found C=44.3; H=2.45; N=6.9%

Calc for $C_{15}H_{10}N_2ClF_3O_4S$ C=44.3; H=2.45; N=6.9%

Example 2

The alpha methylthio benzaldoxime product of Example 1 (2g) in dry tetrahydrofuran (10ml) was added dropwise with stirring under nitrogen at room temperature to a suspension of sodium hydride (0.2g) in tetrahydrofuran (50ml). After an hour, methyl bromoacetate (1g) was added and the reaction mixture refluxed for 1 hour. Ether and water (500ml; 50/50) was added and the organic layer separated, washed and dried. The product was chromatographically purified to yield, as a pale yellow oil, alpha-methylthio 3-(2-chloro-4-trifluoromethylphenoxy)-6-nitro

benzaldehyde, O-methoxycarbonylmethyl oxime.

| | | | | |
|---|---|---|---|---|
| Elemental Analysis: | Found | C=45.7; | H=3.0; | N=5.1% |
| Calc for $C_{18}H_{14}N_2ClF_3O_6S$ | | C=45.2; | H=2.9; | N=5.8% |

Examples 3-21

Following procedures similar to those described in Examples 1 and 2, but replacing the methyl mercaptan reagent of Example 1(f) with the appropriate alternative reactant XH and/or the methyl bromoacetate of Example II with the appropriate alternative reagent Hal Z, additional examples of compounds of the invention were prepared, whose physical properties and chemical analyses are given in Table 1 below. In that Table, the compounds are identified by reference to the identity of the substituents in the following formula:-

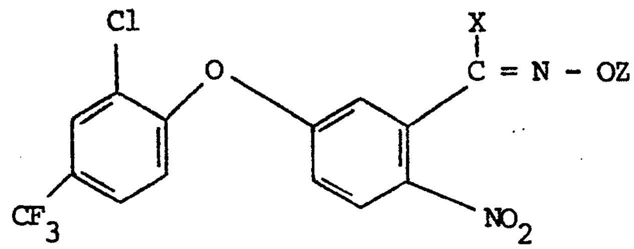

| Example No. | X | Z | m.pt. °C | Analysis | | | | | |
|---|---|---|---|---|---|---|---|---|---|
| | | | | Calc. | | | Found | | |
| | | | | C | H | N | C | H | N |
| 3 | $N(CH_3)_2$ | H | 154-155 | 47.6 | 3.2 | 10.4 | 47.7 | 3.3 | 10.4 |
| 4 | " | $CH_2COOCH_3$ | oil | 47.9 | 3.6 | 8.8 | 47.8 | 3.6 | 8.9 |
| 5 | cyclo $N=(CH_2)_5$ | H | 215-216 | 51.4 | 3.8 | 9.5 | 51.5 | 3.8 | 9.5 |
| 6 | " | $CH_2COOCH_3$ | oil | 51.2 | 4.1 | 8.2 | 50.8 | 4.0 | 7.9 |
| 7 | $SCH_3$ | $COCH_3$ | 112-113 | 45.5 | 2.7 | 6.2 | 45.8 | 2.7 | 6.3 |
| 8 | $SCH_3$ | $CONHCH_3$ | oil | 44.0 | 2.8 | 9.1 | 44.0 | 2.7 | 8.5 |
| 9 | $SCH_3$ | $CH_2$Phenyl | oil | 53.2 | 3.2 | 5.6 | 52.1 | 2.9 | 5.4 |
| 10 | $SCH_3$ | $CH_2CN$ | oil | 45.8 | 2.5 | 9.4 | 45.8 | 2.7 | 8.3 |
| 11 | 1,2,4 triazole | H | 174-175 | 44.9 | 2.1 | 16.4 | 45.7 | 2.1 | 15.9 |
| 12 | $SCOCH_3$ | H | 108 | 44.2 | 2.3 | 6.4 | 44.8 | 2.3 | 6.8 |
| 13 | $SCOCH_3$ | $CH_2COOCH_3$ | oil | 45.0 | 2.8 | 5.5 | 44.8 | 2.8 | 5.9 |
| 14 | $SCH_2COOC_2H_5$ | H | oil | 45.1 | 2.9 | 5.8 | 44.7 | 3.5 | 5.0 |
| 15 | " | $CH_2COOCH_3$ | oil | 45.8 | 3.3 | 5.1 | 45.8 | 3.5 | 5.4 |
| 16 | 1,2,4-triazole | $CH_2COOC_2H_5$ | 145-146 | 45.6 | 2.6 | 14.0 | 45.6 | 2.6 | 13.6 |

| Example No. | X | Z | m.pt. °C | Analysis Calc. C | Calc. H | Calc. N | Found C | Found H | Found N |
|---|---|---|---|---|---|---|---|---|---|
| 17 | S-Phenyl | H | 145 | 51.5 | 2.6 | 6.0 | 51.5 | 2.8 | 5.4 |
| 18 | " | $SO_2CH_3$ | 153-154 | 46.1 | 2.6 | 5.1 | 46.3 | 2.6 | 5.0 |
| 19 | CN | $CH_2COOCH_3$ | 125 | 47.2 | 2.4 | 9.2 | 47.2 | 2.4 | 9.1 |
| 20 | $SCH_3$ | $CH_3$ | oil | 45.7 | 2.9 | 6.6 | 45.8 | 2.9 | 6.5 |
| 21 | $SCH_2COOC_2H_5$ | $CH_3$ | oil | 46.3 | 3.2 | 5.7 | 47.0 | 3.4 | 5.3 |

Example 22 Herbicidal Activity

To evaluate their herbicidal activity, compounds according to the invention were tested using as representative range of plants: maize, Zea mays (Mz); rice, Oryza sativa (R); barnyard grass, Echinochloa crusgalli (BG); oat, Avena sativa (O); linseed, Linum usitatissimum (L); mustard, Sinapsis alba (M); sugar beet, Beta vulgaris (SB) and soya bean, Glycine max (S).

The tests fall into two categories, pre-emergence and post-emergence. The pre-emergence tests involved spraying a liquid formulation of the compound onto the soil in which the seeds of the plant specied mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol/ethylene oxide condensate available under the trade mark TRITON X-155. These acetone solutions were diluted with water and the resulting formulations applied at dosage levels correponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil,

and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in Table II below, in which the compounds are identified by reference to the preceding examples.

Table II

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 1 | | | | 3 | 5 | 6 | 5 | 3 | 5 | 5 | 4 | 6 | 4 | 8 | 9 | 8 | 5 | 3 | 2 | 8 | 3 | 9 | 9 | 9 | |
| | | | | | | | | | 1 | 2 | 2 | 5 | 3 | 8 | 8 | 8 | 5 | 2 | 2 | 8 | 2 | 7 | 7 | 9 | |
| 2 | 6 | 7 | 8 | 6 | 9 | 9 | 9 | 4 | 5 | 7 | 5 | 8 | 7 | 9 | 9 | 9 | 7 | | | 9 | 4 | 9 | 9 | 9 | 4 |
| | | | | | | | | | 1 | 5 | 4 | 6 | 6 | 9 | 9 | 9 | 7 | | | 8 | 3 | 8 | 9 | 9 | 2 |
| 3 | 3 | 5 | 7 | 4 | 6 | 8 | 7 | 2 | 5 | 5 | 4 | 6 | 4 | 8 | 9 | 8 | 6 | 2 | 3 | 8 | 5 | 9 | 9 | 9 | 2 |
| | | | | | | | | | 1 | 3 | 2 | 5 | 4 | 8 | 9 | 7 | 4 | 1 | 2 | 6 | 4 | 7 | 7 | 9 | 2 |
| 4 | 5 | 6 | 8 | 6 | 9 | 9 | 8 | 4 | 5 | 8 | 6 | 9 | 9 | 9 | 9 | 9 | 8 | 3 | 4 | 9 | 8 | 9 | 9 | 9 | 5 |
| | | | | | | | | | 1 | 6 | 4 | 8 | 7 | 9 | 9 | 9 | 7 | 2 | 2 | 8 | 5 | 8 | 9 | 9 | |

Table II (cont'd)

| Compound of Ex. No. | Soil drench 10/kg/ha | | | | | | | | Dosage kg/ha | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 5 | | | | | 5 | 3 | | | 5 | | | 3 | 2 | 5 | 6 | 4 | 3 | | | 4 | | 5 | 7 | 8 | |
| | | | | | | | | | 1 | | | 1 | 1 | 5 | 4 | 3 | 2 | | | 3 | | 3 | 6 | 8 | |
| 6 | 3 | | 2 | | 6 | 5 | 5 | 4 | 5 | 6 | 5 | 8 | 6 | 9 | 9 | 8 | 8 | 5 | 4 | 6 | 3 | 8 | 9 | 9 | |
| | | | | | | | | | 1 | 4 | 3 | 6 | 5 | 8 | 7 | 6 | 6 | | 2 | 2 | 3 | 6 | 6 | 7 | |
| 7 | | | | | | | | | 5 | 4 | 4 | 5 | 4 | 9 | 8 | 6 | 6 | | | 1 | 2 | 3 | 4 | 4 | 2 |
| | | | | | | | | | 1 | 2 | 2 | 3 | 3 | 9 | 7 | 4 | 3 | | | 1 | 1 | | 1 | 3 | 2 |
| 8 | | | | | | | | | 5 | 3 | 3 | 5 | 5 | 9 | 8 | 5 | 6 | | 2 | 3 | 2 | 6 | 7 | 3 | |
| | | | | | | | | | 1 | 1 | 2 | 3 | 2 | 8 | 8 | 4 | 3 | | | 1 | | 2 | 5 | | |
| 9 | 3 | 4 | 4 | 4 | 5 | 6 | | | 5 | 7 | | 7 | 5 | 9 | 9 | 7 | 7 | | | | | | | | |
| | | | | | | | | | 1 | 2 | | 3 | 5 | 8 | 6 | 4 | 6 | | | | | | | | |

Table II (cont'd)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 10 | | 4 | 5 | 4 | 6 | 7 | 6 | | 5 | 6 | 4 | 7 | 6 | 9 | 8 | 9 | 8 | | 3 | 4 | 5 | 4 | 7 | 8 | |
| | | | | | | | | | 1 | 4 | 2 | 4 | 4 | 9 | 7 | 6 | 6 | | 1 | 2 | 4 | 4 | 6 | 7 | |
| 11 | | 5 | 5 | 5 | 5 | 4 | 6 | | 5 | | | 3 | 4 | 9 | 6 | 9 | 5 | | | | 5 | 4 | 7 | 8 | |
| | | | | | | | | | 1 | | | 3 | 3 | 7 | 4 | 4 | 5 | | | | 5 | 2 | 6 | 8 | |
| 12 | | 3 | 5 | 6 | 7 | 5 | 5 | | 5 | 7 | 5 | 8 | 8 | 9 | 8 | 9 | 8 | 3 | 2 | 8 | 8 | 8 | 7 | 9 | 2 |
| | | | | | | | | | 1 | 4 | 4 | 4 | 5 | 9 | 6 | 8 | 7 | | | | | | | | |
| 13 | 3 | 3 | 7 | 6 | 8 | 5 | 8 | | 5 | 7 | 5 | 9 | 9 | 9 | 9 | 9 | 8 | 3 | 3 | 8 | 7 | 8 | 7 | 9 | 5 |
| | | | | | | | | | 1 | 5 | 3 | 6 | 5 | 9 | 9 | 9 | 7 | 2 | 2 | 7 | 6 | 4 | 7 | 8 | 2 |
| 14 | 6 | 7 | 7 | 7 | 7 | 9 | 8 | 6 | 5 | 8 | | 6 | 7 | 9 | 9 | 9 | 9 | | 2 | 4 | 4 | 4 | 9 | 9 | |
| | | | | | | | | | 1 | 4 | | 4 | 4 | 9 | 9 | 7 | 6 | | 1 | 1 | 2 | | 9 | 8 | |

Table II (cont'd)

| Compound of Ex. No. | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | kg/ha | Mz | R | BG | O | L | M | SB | S | Mz | R | BG | O | L | M | SB | S |
| 15 | | | | | | | | | 5 | 6 | | 4 | 4 | 9 | 8 | 6 | 9 | 3 | | | 2 | | 7 | 6 | 2 |
| | | | | | | | | | 1 | 4 | | 3 | 4 | 7 | 6 | 5 | 7 | 2 | | | 1 | | 4 | 6 | 1 |
| 16 | | | | 4 | 7 | 9 | 9 | | 5 | 7 | 4 | 9 | 7 | 9 | 9 | 9 | 5 | 4 | 4 | 6 | 5 | | 9 | 8 | |
| | | | | | | | | | 1 | 4 | 2 | 4 | 6 | 9 | 9 | 7 | 5 | 3 | 2 | 4 | 3 | | 8 | 8 | |
| 17 | | | | | | | | | 5 | 2 | | 1 | | 5 | 3 | 3 | 2 | | | | | | | 7 | |
| | | | | | | | | | 1 | | | | | 5 | 2 | 2 | 2 | | | | | | | 4 | |
| 18 | | | | | | | | | 5 | 5 | 4 | 5 | 4 | 8 | 5 | 6 | 4 | | | | | | | | |
| | | | | | | | | | 1 | 2 | 2 | 4 | 3 | 5 | 3 | 4 | 3 | | | | | | | | |
| 19 | 5 | 7 | 8 | 7 | 7 | 9 | 9 | | 5 | 8 | 7 | 9 | 8 | 9 | 9 | 9 | 7 | 5 | 3 | 7 | 4 | 9 | 9 | 9 | 4 |
| | | | | | | | | | 1 | 6 | 5 | 8 | 7 | 9 | 9 | 9 | 6 | 3 | | 7 | 3 | 8 | 9 | 9 | 2 |

Table II (cont'd)

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 20 | 2 | 3 | 5 | 5 | | 3 | | 4 | 5 | 4 | | 4 | 6 | 9 | 9 | 8 | 5 | | | | 6 | | 6 | 8 | 3 |
| | | | | | | | | | 1 | 2 | | 2 | 4 | 8 | 9 | 7 | 4 | | | | 4 | | 2 | 7 | |
| 21 | | | | | | | | | 5 | 8 | 7 | 9 | 8 | 9 | 9 | 9 | 9 | 7 | 6 | 9 | 7 | 9 | 9 | 9 | 8 |
| | | | | | | | | | 1 | 6 | 6 | 8 | 6 | 9 | 9 | 9 | 8 | 4 | 4 | 8 | 4 | 9 | 9 | 9 | 7 |

K 1963

CLAIMS

1. Oximino diphenyl ether derivatives having the general formula I:-

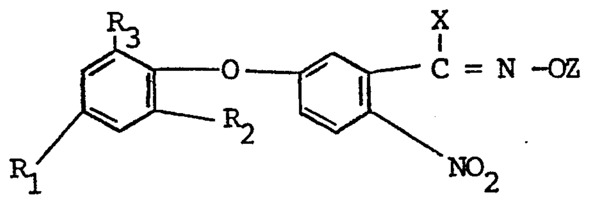

(I)

wherein $R_1$ represents a halogen atom or a haloalkyl group; $R_2$ and $R_3$, which may be the same or different, each independently represents a hydrogen or halogen atom or a nitro, cyano or haloalkyl group;
X represents a cyano or triazole group or a group $SR_4$ or $NR_5R_6$ in which $R_4$ represents an optionally substituted alkyl, aryl, aralkyl, alkoxycarbonylalkyl or acyl group; $R_5$ represents an optionally substituted alkyl or aralkyl group and $R_6$ represents an optionally substituted alkyl, aralkyl or alkoxy group, or the groups $R_5$ and $R_6$ together jointly represent an alkylene group;
and Z represents a hydrogen atom or an optionally substituted alkyl, cycloalkyl, alkenyl, aralkyl, alkoxycarbonylalkyl, or acyl group.

2. Compounds as claimed in claim 1 wherein $R_1$ represents a trifluoromethyl group, $R_2$ represents a chlorine atom and $R_3$ represents a hydrogen atom.

3. Compounds as claimed in claim 1 or 2 wherein X represents a cyano or triazole group or the group $SR_4$ wherein $R_4$ is an alkyl group containing up to 4 carbon atoms, a phenyl

group, or alkanoyl or alkoxycarbonylalkyl containing up to 8 carbon atoms, or a group $NR_5R_6$ wherein $R_5$ and $R_6$ each represents an alkyl group containing up to 4 carbon atoms, or $R_5$ and $R_6$ together jointly represent an alkylene group containing from 3 to 8 carbon atoms.

4. Compounds as claimed in Claim 1, 2 or 3 wherein Z represents a hydrogen atom, an alkyl group containing up to 4 carbon atoms optionally substituted by a cyano or phenyl group, an alkanoyl, alkylcarbamoyl or alkylsulphonyl group of up to 6 carbon atoms, or an alkoxycarbonylalkyl group containing up to 8 carbon atoms.

5. Process for the preparation of a compound of the general formula I as defined in claim 1, which comprises reacting a halo aldoxime of formula II:

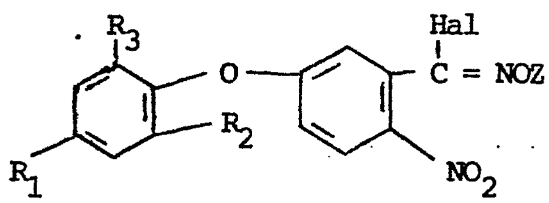

(II)

with a compound of formula XH, wherein $R_1$, $R_2$, $R_3$, X and Z are as defined in claim 1 and Hal represents halogen, in the presence of a base, followed, when Z is hydrogen and the desired product is one wherein Z has one of the alternative significations, by reaction with a compound of formula Hal Z in the presence of an alkali metal hydride.

6. Process as claimed in claim 5 wherein the base is triethylamine and the reaction is carried out in an organic solvent.

7. Process as claimed in claim 5 or 6 wherein the final reaction with Hal Z, when applied, is carried in a polar organic solvent.

8. A compound as defined in claim 1, whenever prepared by a process as claimed in any one of claims 5-7.

9. Herbicidal composition, which comprises a compound as claimed in any of claims 1 to 4 and 8, together with a carrier.

10. Method of combating undesired plant growth at locus, which comprises treating the locus with a compound as claimed in any one of claims 1 to 4 and 8, or a composition as claimed in claim 9.
11. The use of a compound as claimed in any one of claims 1 to 4 and 8 as a herbicide.